(19) Europäisches Patentamt
European Patent Office
Office européen des brevets

(11) Publication number: 0 407 651 A2

# (12) EUROPEAN PATENT APPLICATION

(21) Application number: 89115998.0

(51) Int. Cl.[5]: A61K 31/52

(22) Date of filing: 30.08.89

Claims for the following Contracting State: GR.

(30) Priority: 10.07.89 ES 8902422

(43) Date of publication of application:
16.01.91 Bulletin 91/03

(84) Designated Contracting States:
AT BE CH DE FR GB GR IT LI LU NL SE

(71) Applicant: J. URIACH & CIA. S.A..A.
Degà Bahi, 59-67
E-08026 Barcelona(ES)

(72) Inventor: Bartroli, Javier
Vives i Tut 55, 2 ,2a
E-08034 Barcelona(ES)
Inventor: G mez, Luis
Gomis 51, 1 ,4a
E-08023 Barcelona(ES)
Inventor: Garcia-Rafanell, Julián
Rosell 416, Atco. 2a
E-08025 Barcelona(ES)
Inventor: Forn, Javier
Paseo de los Tilos 25
E-08034 Barcelona(ES)

(74) Representative: Zumstein, Fritz, Dr. et al
Zumstein & Klingseisen Patentanwälte
Bräuhausstrasse 4e 4
D-8000 München 2(DE)

## (54) Use of 1,3-diisobutyl-8-methylxanthine as a bronchodilator and antiallergy agent.

(57) The invention relates to the use of 1,3-diisobutyl-8-methylxanthine, or its pharmaceutically acceptable salts, for the treatment or prophylaxis of acute or chronic obstructive airways disease and allergic asthma. The invention also relates to a pharmaceutical composition of an aerosol containing 1,3-diisobutyl-8-methylxanthine, ethanol, sorbitan oleate and one or more freons.

EP 0 407 651 A2

# USE OF 1,3-DIISOBUTYL-8-METHYLXANTHINE AS A BRONCHODILATOR AND ANTIALLERGY AGENT

## FIELD OF THE INVENTION

The present invention relates to a new therapeutic use for 1,3-diisobutyl-8-methylxanthine or its pharmaceutically acceptable salts.

More particularly, the invention relates to the use of 1,3-diisobutyl-8-methylxanthine as a bronchodilator and antiallergy agent by inhalation administration.

## BRIEF DESCRIPTION OF THE PRIOR ART

Bronchial asthma is a disease of multifactorial nature difficult to restrict to an unique description. However, a common feature to all forms of this disease is an enhanced bronchoconstrictory responsiveness to non-specific stimulation. Many types of allergic reactions in humans consist of an antigen-antibody reaction which leads to the release from mast cells of pharmacologically active agents, such as histamine and slow reacting substance of anaphylaxis (SRS). These mediators cause broncho-constriction and, therefore, bronchial asthma of the exogenous type may be considered as a form of allergy.

A good approach in the therapy of bronchial asthma is provided by the use of drugs having a dual bronchodilator and antiallergy activity. These agents can be useful not only in the treatment of acute asthma attacks, but also in the prevention of allergic asthma.

The effectiveness of xanthine derivatives in the treatment of these disorders is widely referenced in the literature. However, up to the present time, only few and controversial works have been published on their action when given as an aerosol formulation (*Chest*, **1981**,*79*,4; *Bull. Int. Union against Tuberculosis*, **1979**, *54*,182). Thus, whereas in some trials aerosolized theophylline has been reported to be succesful (*J.Tenn.Med.Ass.* **1966**, *59*,239), in others has given a negative response (*Helv.Med.Acta*, **1969**,*34*,517; *Chest*, **1976**, *69*, 718). Another well known xanthine, diphylline or 7-[2,3-dihydroxypropyl]theophylline, has been used as a bonchodilator agent for decades as a parenteral and oral preparation. However, when administered as an aerosol preparation, it has been reported to be not only an inneffective bronchodilator, but even to induce bronchospasm in human asthmatics (*J.Med.Clin.Exp.Theor.*, **1982**, *13*, 275). We have found that aerosolized 1,3-diisobutyl-8-methylxanthine is much more active than theophylline.

## DESCRIPTION OF THE INVENTION

According to the present invention, we provide 1,3-diisobutyl-8-methylxanthine, i.e. the compound of formula **I**

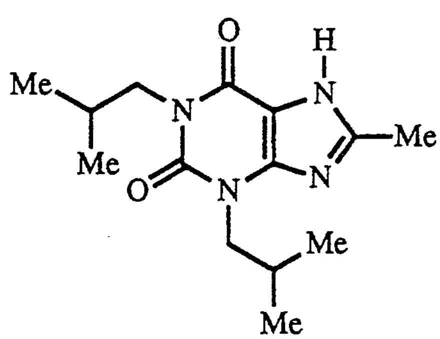

**I**

and pharmaceutically acceptable salts thereof, for use in the treatment or prophylaxis of acute or chronic

obstructive airways disease and allergic asthma.

According to a further feature of the present invention, we provide the use of 1,3-diisobutyl-8-methylxanthine or a pharmaceutically acceptable salt thereof in the manufature of a medicament for the treatment or prophylaxis of acute or chronic obstructive airways disease and allergic asthma.

According to a further feature of the present invention, we provide the use of 1,3-diisobutyl-8-methylxanthine or a pharmaceutically acceptable salt thereof in the manufature of an aerosol formulation for the treatment or prophylaxis of acute or chronic obstructive airways disease and allergic asthma.

Pharmaceutically acceptable salts which are especially preferred for therapeutic use are salts of alkali metals such as sodium and potassium, and alkaline earth metals such as calcium and magnesium.

Theophylline, or 1,3-dimethylxanthine, is a widely used drug in the therapy of bronchospastic diseases. However, it shows both narrow therapeutical range and a very irregular pharmacokinetic behavior when administered to humans. This, together with its important side effects, such as nausea, vomiting and mild central nervous manifestations (including irritability or confusion), observed after oral administration, constitute an important drawback in theophylline therapy. In order to overcome these problems new pharmacologically active xanthine drivatives have been developed. Among them 1-methyl-3-isobutylxanthine (IBMX), although structurally similar to theophylline, exhibits unexpectedly high *in vivo* and *in vitro* bronchodilator activity. Unfortunately, this compound has a high level of toxicity that impedes its therapeutical use in humans.

The need of new drugs having a suitable activity and a low toxicity in this field has led to the preparation of a series of xanthine derivatives with different substituents on the purine ring. Many of these compounds have been reported in the literature.

Thus, several previous reports describe compounds structurally similar to 1,3-diisobutyl-8-methylxanthine. However, either they have been claimed as having a different therapeutic use, or they show a low bronchodilator activity, or they possess a disfavorable kinetic behavior. For example, patent US 2,729,643 describes a series of xanthines substituted at positions 1,3 and 8 with alkyl groups, useful as diuretic agents. In said patent, 1,3-diisobutyl-8-methylxanthine is metioned, but no comment about its bronchodilator activity is made, nor any physical data is given.

It can be considered that some of the compounds described in *Biochem.Z.* **1925**, *163,*13; *Ann.Chim. (Roma)* **1955**, *45,* 983; *Farmaco (Pavia)* **1957**, *12,* 1016;*J.Pharmacol.Exptl.Therap.* **1940**,*69,* 45; and *J.Am.Chem.Soc* **1953**, *75,* 114 are structurally similar to 1,3-diisobutyl-8-methylxanthine, but again, in none of those works it is suggested the use of those compounds as bronchodilator agents.

In *J.Pharmacol.* **1961**, *17,* 196, and *J.Med.Chem.* **1971**, *14* 1202, certain di and trialkyl derivatives are described as bronchodilator agents. However, 1,3-diisobutyl-8-methylxanthine is not mentioned.

Patent DE 2,713,389 discloses a series of xanthines with bronchodilator activity having alkyl substituents in 1,3 and 8 and a hydrogen atom or an alkoxycarbonyl group m position 7. As before, 1,3-diisobutyl-8-methylxanthine is not mentioned.

Finally, patent application EP 39,780 describes a series of 1,3-di and 1,3,8-trialkylxanthines, including 1,3-diisobutyl-8-methylxanthine, having neuroleptic properties. Accordingly, some of the compounds described therein are said to have, in addition to the neuroleptic activity, some diuretic, antiallergy, bronchodilator and antihistamine activities, but showing minimum effective doses much higher than the effective doses ($ED_{50}$) for the neuroleptic effect. In any case, said patent application neither specifically describes 1,3-diisobutyl-8-methylxanthine as having a bronchodilator activity, nor it mentiones its use in the treatment of bronchospastic diseases.

The pharmaceutical compositions of the present invention for the treatment of bronchospastic and allergy disorders may be given by inhalation as an aerosol. The compositions are administered at dose levels of 1,3-diisobutyl-8-methylxanthine, or a pharmaceutically acceptable salt thereof, of about 0.002 to 0.2 mg per Kg, preferably of about 0.01 to 0.1 mg per Kg, and most preferably of about 0.025 to 0.05 mg per Kg of human body weight per day, and are used in unit dosage or multidosage form, administered one to twelve times per day in an amount of 0.1 to 1 mg per aerosol puff.

Formulations of the present invention suitable for aerosol administration can be prepared by dissolving or suspending 1,3-diisobutyl-8-methylxanthine (preferably 0.1 to 1.0% of the total weight) in a suitable pharmaceutically acceptable solvent, for example, ethyl alcohol, propylene glycol, dipropylene glycol, etc (preferably 25-35% of the total weight), or combinations of such solvents, and mixed with a pharmaceutically acceptable propellant, such as freons 11, 12 and/or 114 (preferably 60-99% of the total weight), and, if necessary, a pharmaceutically acceptable tensoactive agent, such as sorbitan trioleate, cetylpyridinium chloride, or isopropyl miristate (preferably 0.2-1.0% of the total weight). Such aerosol compositions are packaged for use in a pressurized container fitted with an aerosol valve suitable for release of the pressurized composition. Preferably, the aerosol valve is a metered valve, that is one which on activation

releases a predetermined effective dose of the aerosol composition.

The results of the biological tests reveal 1,3-diisobutyl-8-methyl-xanthine to be a potent bronchodilator agent, with an activity 300x fold higher than theophylline in the isolated guinea pig trachea relaxation assay. *In vivo,* 1,3-diisobutyl-8-methylxanthine is much more potent than theophylline against histamine-induced bronchospasm both in anesthetized and conscious guinea pigs. Additionally, 1,3-diisobutyl-8-methylxanthine shows an outstanding antibronchoconstrictory activity following administration by inhalation to conscious guinea pigs. This latter property makes 1,3-diisobutyl-8-methylxanthine suitable for its manufacture as an aerosol formulation, in contrast to commercialized xanthine derivatives, e.g. theophylline, aminophylline and diphylline, whose poor bronchodilator activity and other shortcomings, e.g. taste disturbances and local irritance, impede their local application to the airways.

Furthermore, 1,3-diisobutyl-8-methylxanthine exhibits a marked antiallergy activity as demonstrated by its ability to inhibit the release of histamine induced by compound 48/80 (purchased from Sigma Chem. Co.) in rat peritoneal mast cell preparations and the passive cutaneous anaphylaxis reaction (PCA) in the rat, and particularly by its ability to protect sensitized guinea pigs against allergic (egg-albumin) bronchospasm with a higher potency than the reference compound theophylline.

Both, the potent bronchodilator and antibronchospatic activities, and the antianaphylactic properties of 1,3-diisobutyl-8-methylxanthine make this compound suitable for the treatment of asthma.

The acute oral toxicity of 1,3-diisobutyl-8-methylxanthine in both sexes rats is 50 mg/Kg, a much higher value than the active doses given by inhalation (<0.5 mg/animal). This indicates that 1,3-diisobutyl-8-methylxanthine posseses a high safety margin when used by inhalation in the local treatment of bronchial and allergic asthma.

The following Examples illustrate the present invention:

**EXAMPLE 1: FORMULATIONS (in % weight)**

| **Formulation A** | |
|---|---|
| 1,3-diisobutyl-8-methylxanthine | 0.5 |
| Sorbitan trioleate | 0.5 |
| Freon 12 | 64.5 |
| Freon 11 | 18.5 |
| Freon 114 | 16.0 |
| TOTAL | 100% |

| **Formulation B** | |
|---|---|
| 1,3-diisobutyl-8-methylxanthine | 0.5 |
| Ethyl alcohol | 30.5 |
| Sorbitan trioleate | 0.5 |
| Freon 12 | 54.8 |
| Freon 114 | 13.7 |
| TOTAL | 100% |

**EXAMPLE 2: BRONCHODILATOR ACTIVITY**

**Test 1: Isolated guinea pig trachea**

This *in vitro* test was carried out according to the method of Emmerson and Mackay (*J. Pharm.*

*Pharmacol.* **1979**, *31*, 798). The trachea was excised from guinea pigs, placed in Krebs Henseleit's solution at 37° C and gassed with 95% $O_2$ and 5% $CO_2$. The trachea strip was mounted in an organ bath and attached to a tension transducer preloaded with 0.5 mg. Test compounds were cumulatively added to the organ bath and concentrations causing 50% relaxation of the spontaneous tonus of the tracheal tissue ($EC_{50}$) were calculated (Table 1). The relaxation induced by 1 mcM isoprenaline was taken as 100%.

**Test 2: Histamine-induced bronchospasm in anaesthetized guinea pigs (Konzett and Rössler)**

This test was performed in anaesthetized animals according to the method of Konzett and Rössler (*Arch. Exp. Pathol.Pharmacol.* **1940**, *195*, 71). The activity was determined by calculating the dose of the active compound required to produce 50% inhibition of the bronchospasm ($ID_{50}$) (Table 1). Histamine was given intravenously at 10 mcg/Kg, before and 10, 30 and 60 minutes after intraperitoneal administration of test compounds. Maximum inhibitory effects were taken for calculation of $ID_{50}$ values.

**Test 3: Histamine aerosol-induced bronchospasm**

This test is based on the methods of Van P+roosdif-Hartzema *(Inter. Ppharmacodynamic* **1958**, *115*, 322) and Loew (*J. Pharmac.Exp.Ther.* **1945**, *83*, 120) with slight modifications. Thus, conscious guinea pigs were subjected to inhalation of histamine (0.2% aqueous solution) nebulized by an ultrasonic device (DeVilvis™, Ultra-Neb 99) during 30 seconds in a cylindric plastic chamber of 15 liters of volume, at a rate of 1 mL/min. Test compounds were given orally, one hour before the challenge of histamine, and the dose needed to protect from death 50% of the animals was determined ($PD_{50}$) (Table 1).

Using the same model, test compounds were also given by inhalation. They were dissolved in ethanol at different concentrations (0.45 mM to 28mM) and aerosolized by the ultrasonic nebulizer mentioned above at a rate of 1 mL/min, during 2 minutes. Three minutes later, the animals were subjected to the challenge of histamine and $PC_{50}$ values were determined (Table 1). Controls were subjected to inhalation of the vehicle (ethanol). $PC_{50}$ value is defined as the concentration of test compound in the ethanolic solution that protects 50% of animals from lethality when nebulized as indicated above.

Finally, studies have been made subjecting the animals to inhalation of one or more puffs of 5 mg/mL of 1,3-diisobutyl-8-methylxanthine (UR-6032) solution formulation B (see Example 1). Animals were forced to inhale UR-6032 for 5 minutes into the cylindric plastic chamber before the challenge with ultrasonic nebulized histamine (see above) and protection from death with respect to controls was determined (Table 2).

Table 1.

| **Bronchodilator activity** | | | |
|---|---|---|---|
| Test compound | Guinea pig trachea $EC_{50}$ (mcM) | Histamine bronchospasm | |
| | | Hist. i.v. $ID_{50}$ (mg/Kg,i.p.) | Hist. aerosol $^{a}PD_{50}$ (oral) $^{b}PC_{50}$ (inhal) |
| UR-6032 | 0.1 | 0.36 | $^{a}$ 1 mg/Kg $^{b}$ 0.5 mM |
| Theophylline | 31 | 4.90 | $^{a}$>100 mg/Kg $^{b}$ 25 mM |
| IBMX | 1.1 | 2.50 | $^{b}$ 15-20 mM |

Table 2

| Bronchodilator activity: histamine aerosol-induced bronchospasm in guinea pigs | | | |
|---|---|---|---|
| Test compound | Number of puffs | Protection from death | |
| | | [a]n/N | % |
| Control | - | 0/7 | 0 |
| [b]UR-6032 | 1 | 4/5 | 80 |
| | 2 | 3/3 | 100 |
| | 3 | 3/3 | 100 |

[a]n/N: number of survivors (n) versus total number of animals(N)
[b]UR-6032 : formulation B (0.5 mg/mL, 0.5 mg/puff)

## EXAMPLE 3: ANTIALLERGY ACTIVITY

### Test 4: Histamine release induced by compound 48/80 in rat mast cell preparations.

Anaesthetized rats were bled by cardiac puncture and then 10 mL of Tyrode's-gel solution (0.1% gelatine in Tyrode's buffer, pH 7.2) containing 50 mcg heparin was injected into the peritoneal cavity. Alter a gentle massage of the abdomen, the peritoneal cell suspension was harvested. Mast cells were suspended in Tyrode's solution ($10^6$ cells/mL). Aliquots of 0.2 mL of the mast cell suspension were incubated at 37°C for 20 minutes with 10 mcL of compound 48/80 ($10^{-6}$ M final concentration), a potent histamine release inducer. The reaction was stopped by introducing the tubes in a ice-ethanol bath. The cells were sedi-mented by centrifugation and the histamine in the supernatant was determined by the fluorimetric method of May (***J. Allergy***, **1970**, *46*, 12). The sedimented pellets were treated with 0.1 N HCl at 100°C to liberate the non-released histamine and to determine it by the same method of May. Test compounds were added to the incubation medium dissolved in Tyrode's buffer at different concentrations (final volume: 1 mL). Control samples contained the same volume of Tyrode's buffer. The histamine release in the control samples was taken as 100%, and the percentage of the histamine release inhibition of test samples was calculated with respect to controls. (Table 3).

### Test 4: Egg albumin-induced bronchospasm in sensitized guinea pigs.

The experimental procedure of Konzett and Rössler was followed (*Arch. Exp. Pathol.Pharmacol.* **1940**,*195,* 71). A single intravenous injection of 1 mL/Kg of egg albumin (0.06%, w/v) was given to sensitized animals one minute after intravenous administration of UR-6032 at different concentrations. $ID_{50}$ values are reported in Table 3.

### Test 5: Passive cutaneous anaphylaxis reaction (PCA) in the rat

The animals were passively sensitized by intradermal injection of 0.1 mL of five-fold diluted rat reagenic antiserum. Injections were made at 4 locations on the shaved back skin and 48 h later the test drug was administered intraperitoneally. Thirty minutes later, the animals were challenged by intravenous injection of egg albumin and Evans' blue dye. The dermal reaction was measured colorimetrically by the method of Harata (*J.Pharm.Pharmac.,* **1971**, *23,* 218) as the extent of the blueing reaction at the sensitized intradermal injection site, and the percent inhibition values with respect to controls were calculated (Table 3).

Table 3.

| Antiallergy activity | | | |
|---|---|---|---|
| Test compound | Histamine release % inhib. at 1 mM | PCA % inhibition at 2.5 mg/Kg,i.p. | Egg albumin bronchospasm $ID_{50}$ (mg/Kg,i.p.) |
| UR-6032 | 56 | 39 | 0.5-1 |
| Theophylline | 25 | 8 | - |
| IBMX | 37 | 86 | - |

**EXAMPLE 4: PREPARATION OF 1,3-DIISOBUTYL-8-METHYLXANTHINE**

**A) N,N′-diisobutyl-N-cyanoacetylurea**

To 300 mL of acetic anhydride was added 146.4 g (0.85 mol) of N, N′-diisobutylurea and 83.3 g (0.98 mol) of cyanoacetic acid. The mixture was stirred at 65-70 °C during 2 hours, and then the excess of acetic anhydride was distilled off under reduced pressure at a temperature not higher than 60 °C. The oily residue was directly used in the next step, without further purification. Yield about 203 g (100%).

**B) 1,3-Diisobutyl-4-aminouracil.**

To the oily residue containing 203 g (about 0.85 mol) of N,N′-diisobutyl-N-cyanoacetylurea, was added with vigorous stirring 340 ml of 10% aqueous NaOH at 40 °C. Upon solution of the urea, a spontaneous increase of the temperature (65-70 °C) was produced, and the formed uracil started to precipitate. The stirring was maintained without heating one additional hour, and then the reaction mixture was cooled in an ice bath, in order to complete precipitation. The precipitate was collected by suction filtration, washed thoroughly with cold water, and dried at 45 °C under vacuum. The crude product was a pale greyish-yellow solid, that could be directly used without further purification. Yield 183 g (90%). Melting point 95-96 °C. An analytical sample was obtained by a two-fold recrystallization from ethyl acetate, as a white crystalline solid: mp: 134-136 °C; IR (KBr) $\nu$ 3349, 3208, 2959, 2868, 1694, 1615, 1490, 1405, 1278, 787, 554 $cm^{-1}$; $^1H$ RMN (60MHz, DMSO-$d_6$) $\delta$ (TMS) 6.58 (br s, 2H, $NH_2$), 4.55 (s, 1H, =CH), 3.55 (d, J=7Hz, 2H, $NCH_2$), 3.41 (d, J=7Hz, 2H, $NCH_2$), 1.85 (m, 2H, 2CHMe₂), 0.65 (d, J=7Hz, 6H, CH**Me**$_2$), 0.60 (d, J=7Hz, 6H,CH$Me_2$). Analysis calculated for $C_{12}H_{21}N_3O_2$: C 60.23%; H 8.84%; N 17.56%. Found: C 60.29%; H 9.00%; N 17.46%.

**C) 1,3-Diisobutyl-4-amino-5-nitrosouracil.**

To a cooled (5 °C) stirred mixture containing 183 g (0.76 mol) of 1,3-diisobutyl-4-aminouracil in 1350 ml of water and 128 ml of acetic acid was added 58 g (0.84 mol) of $NaNO_2$ in small portions. A deep purple colour, characteristic of the nitroso derivative, appeared immediately, and stirring was continued at room temperature for two hours. The mixture was then cooled in an ice bath, and stirred for one additional hour. The solid was filtered off, washed with cold water, and dried at 45 °C under vacuum. The crude product was pure enough for further use. About 190 g (93% yield) of a fine crystalline, deep purple solid was obtained. mp: 222-224 °C; IR (KBr) $\nu$ 3198, 2958, 2871, 1720, 1673, 1511, 1409, 1235, 1114,753 $cm^{-1}$; $^1H$ RMN (60 MHz, DMSO-$d_6$) $\delta$ (TMS) 3.66 (d, J=7Hz, 4H, $NCH_2$), 1.90 (m,2H,2C**H**$Me_2$), 0.75 (d, J=6Hz, 12H,2CH**Me**$_2$). Analysis calculated for $C_{12}H_{20}N_4O_3$: C 53.72%; H 7,51%, N 20.88%. Found: C53.90%; H7.72%; N20.63%.

**D) 1,3-Diisobutyl-4,5-diaminouracil**

To a stirred mixture containing 191 g (0.71 mol) of 1,3-diisobutyl-4-amino-5-nitrosouracil in 1000 ml of 25% aqueous ammonia, was added 435 g (2.5 mol) of sodium dithionite in small portions, whereby the temperature raised to 35-36 °C, and the purple colour disappeared gradually. When no more increase of temperature was observed, the reaction mixture was externally heated at 50 °C for one additional hour. At this moment, the colour had almost disappeared, and the mixture was then allowed to stand overnight at room temperature, in order to complete the reaction (total disappearance of the colour). The 1,3-diisobutyl-4,5-diaminouracil precipitated from the ammonia solution, and the reaction mixture was then cooled in an ice-bath to achieve the total precipitation. Finally, the solid was filtered off, thoroughly washed with cold water, and dried at room temperature under vacuum over NaOH pellets. The crude product was used without further purification. The yield was 159 g (88%) of a brownish-grey solid, showing a not-well-defined melting point at about 110-130 °C.

IR (KBr) $\nu$ 3350, 3219, 2959, 2871, 1688, 1646, 1603, 1489, 1273, 1100,762 $cm^{-1}$; $^1H$ NMR (60 MHz, $CDCl_3$) $\delta$ (TMS) 5.12 (br s, 2H, $NH_2$), 3.81 (d, J = 7Hz, 2H, $NCH_2$), 3.73 (d, J = 7Hz, 2H, $NCH_2$), 2.25 (m, 2H, 2C**H**$Me_2$), 1.02 (d, J = 7Hz, 6H, CH**Me**$_2$), 0.90 (d, J = 7Hz, 6H, CH**Me**$_2$).

Analysis calculated for $C_{12}H_{22}N_4O_2$: C 56.67%; H 8.72%; N 22.03%. Found: C 56.34%; H 8.49%; N 21.86%.

**E) 1,3-Diisobutyl-4-amino-5-acetylaminouracil.**

158 g (0.62 mol) of 1,3-diisobutyl-4,5-diaminouracil was mixed with vigorous stirring with 500 ml (8.74 mol) of acetic acid. The mixture was refluxed for two hours, resulting in a clear solution. The excess of acetic acid was distilled off under reduced pressure. To the residue was added 50 ml of absolute ethanol, and evaporated *in vacuo*. This was repeated 3 times, in order to completely eliminate the excess of acetic acid. The residue was a brown coloured paste, which was used in the next step without further purification. Yield:157 g (about 85%). An analytical sample was prepared by recrystallization from methanol, yielding a while crystalline solid.

mp: 128-130 °C; IR (KBr) $\nu$ 3399, 3327, 3241, 2958, 2870, 1713, 1667, 1637, 1552,1498,1284,818,514 $cm^{-1}$; $^1H$ NMR (60 MHz, DMSO-$d_6$) $\delta$ (TMS) 8.30 (m, 1H, NHC = O), 6.67 (m, 2H, $NH_2$), 3.62 (m, 4H, $2NCH_2$), 1.97 (m, 2H, 2C**H**$Me_2$), 1.83 (br s, 3H, $CH_3C = O$), 0.80 (m, 12H, 2CH**Me**$_2$).

Analysis calculated for $C_{14}H_{24}N_4O_3$: C 56.74%; H 8.16%; N 18.90%. Found: C 56.87%; H 8.41%; N 18.66%.

**F) 1,3-Diisobutyl-8-methylxanthine.**

1,3-Diisobutyl-4-amino-5-acetylaminouracil (157 g 0.53 mol), was suspended with vigorous stirring in 477 ml of 15% aqueous NaOH, and heated to reflux for 45 minutes, whereby most of the solid was dissolved. The reaction mixture was allowed to cool to 40 °C with stirring, and then it was filtered by suction, in order to eliminate the non-soluble impurities. To the resulting clear solution was added a little decolourizing carbon, and filtered again. Then, the xanthine was precipitated by the addition of hydrochloric acid until pH 2-3. The suspension was cooled in an ice bath to complete precipitation. Finally, the solid was filtered off, thoroughly washed with ice-cold water, and dried under vacuum at 50 °C over NaOH pellets. The yield was 125 g (85%) of a pale yellowish-white solid melting at 234-236 °C. The crude product could be further used without purification. An analytical sample was prepared by recrystallization from methanol, yielding white needle-like crystals.

mp 240.9-241.0 °C; IR (KBr) $\nu$ 3144, 3088, 3045, 2959, 2867, 1707, 1646, 1558, 1503, 1284, 1091, 1014, 816, 764 $cm^{-1}$; $^1H$ NMR (60 MHz, $CDCl_3$) $\delta$ (TMS) 4.02 (d, J = 8Hz, 2H, $NCH_2$), 4.00 (d, J = 8Hz, 2H, $NCH_2$), 2.60 (s, 3H, $= CCH_3$), 2.35 (m, 2H, 2C**H**$Me_2$), 0.96 (d, J = 7Hz, 12H, 2CH**Me**$_2$).

Analysis calculated for $C_{14}H_{22}N_4O_2$: C 60.41%; H 7.97%; N 20.13%. Found: 60.17%; H 8.42%; N 19.90%.

## Claims

1. Use of 1,3-diisobutyl-8-methylxanthine, i.e. of formula **I**

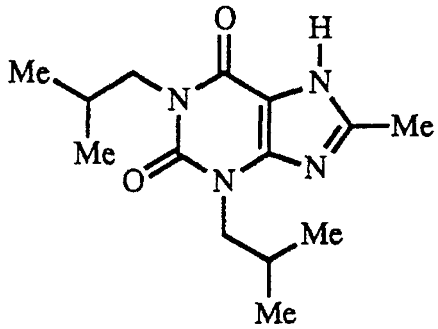

I

or a pharmaceutically acceptable salt thereof, for the manufacture of a pharmaceutical composition for the treatment or prophylaxis of acute or chronic obstructive airways disease and allergic asthma.

2. Use of the compound of formula **I** or a pharmaceutically acceptable salt thereof, according to claim 1 wherein the said pharmaceutical composition is adapted for the inhalation administration.

3. Use of the compound of formula **I** or a pharmaceutically acceptable salt thereof, according to claim 1 wherein the said pharmaceutical composition is in the form of an aerosol.

4. Use of the compound of formula **I** or a pharmaceutically acceptable salt thereof, according to claim 1 wherein the said pharmaceutical composition is administered in an amount of 0.1 to 1 mg of compound of formula **I** per unit dose, one to twelve times per day.

5.- A pharmaceutical composition containing a therapeutically active amount of 1,3-diisobutyl-8-methylxanthine, or a pharmaceutically acceptable salt thereof, a pharmaceutically acceptable solvent such as ethanol, a pharmaceutically acceptable tensoactive agent such as sorbitan trioleate, and one or more pharmaceutically acceptable freons, such as freons 11, 12 and 114, used for the treatment or prophylaxis of acute or chronic obstructive airways disease and allergic asthma.

CLAIMS FOR THE FOLLOWING CONTRACTING STATE: GR

1. A process for preparing an aerosol containing 1,3-diisobutyl-8-methylxanthine, *ie,* a compound of formula **I**

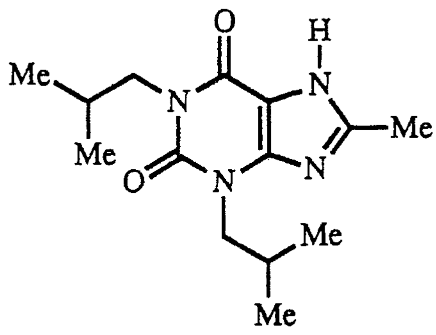

I

or a pharmaceutically acceptable salt thereof, useful for the treatment or prophylaxis of acute or chronic obstructive airways disease and allergic asthma, consisting in admixing a therapeutically active amount of 1,3-diisobutyl-8-methylxanthine, or a pharmaceutically acceptable salt thereof, a pharmaceutically acceptable solvent such as ethanol, a pharmaceutically acceptable tensoactive agent such, as sorbitan trioleate, and one or more pharmaceutically acceptable freons, such as freons 11,12 and 114.

2. A process according to claim 1 wherein said aerosol is administered in an amount equivalent to 0.1 to 1 mg of compound of formula **I** per unit dose, one to twelve times per day.